# EUROPÄISCHE PATENTSCHRIFT

(11) **EP 3 751 289 B1**
(45) Veröffentlichungstag und Bekanntmachung des Hinweises auf die Patenterteilung: **06.05.2026**
(21) Anmeldenummer: 19179863.6
(22) Anmeldetag: 13.06.2019
(51) Int. Cl.: G01N 35/10

(54) **VERFAHREN ZUR OPTISCHEN ÜBERWACHUNG EINER DOSIERUNG EINER ZU PIPETTIERENDEN FLÜSSIGKEIT**
METHOD FOR OPTICALLY MONITORING A DOSING OF A LIQUID TO BE PIPETTED
PROCÉDÉ DE SURVEILLANCE OPTIQUE D'UN DOSAGE D'UN LIQUIDE À PIPETTER

(43) Veröffentlichungstag der Anmeldung: 16.12.2020
(73) Patentinhaber: Siemens Healthcare Diagnostics Products GmbH, 35041 Marburg (DE)
(72) Erfinder: Daume, Dominik Andreas, 69121 Heidelberg (DE); Michels, Thorsten, 64521 Gross-Gerau (DE)
(74) Vertreter: Siemens Healthineers Patent Attorneys

(56) Entgegenhaltungen:
- US-A- 5 601 980
- US-A1- 2018 348 247
- US-A1- 2019 151 840

## Beschreibung

Die Erfindung betrifft ein Verfahren zur optischen Überwachung einer Dosierung einer zu pipettierenden Flüssigkeit für ein automatisches Analysegerät. Zahlreiche Nachweis- und Analyseverfahren zur Bestimmung physiologischer Parameter in Körperflüssigkeitsproben oder anderen biologischen Proben werden heute automatisiert in großer Anzahl in automatischen Analysegeräten, auch so genannten in vitro-Diagnostiksystemen, durchgeführt.

Heutige Analysegeräte sind in der Lage, eine Vielzahl von Nachweisreaktionen und Analysen mit einer Probe durchzuführen. Um eine Vielzahl von Untersuchungen automatisiert durchführen zu können, sind diverse Vorrichtungen zum räumlichen Transfer von Messzellen, Reaktionsbehältern und Reagenzbehältern erforderlich, wie z.B. Transferarme mit Greiffunktion, Transportbänder oder drehbare Transporträder, sowie Vorrichtungen zum Transfer von Flüssigkeiten, wie z.B. Pipettiervorrichtungen. Die Geräte umfassen eine Steuereinheit, die mittels entsprechender Software dazu in der Lage ist, die Arbeitsschritte für die gewünschten Analysen weitgehend selbstständig zu planen und abzuarbeiten.

Viele der in derartigen automatisiert arbeitenden Analysegeräten verwendeten Analyseverfahren beruhen auf optischen Methoden. Diese Verfahren ermöglichen den qualitativen und quantitativen Nachweis von Analyten, d.h. der nachzuweisenden oder zu bestimmenden Substanzen in Proben. Die Bestimmung klinisch relevanter Parameter, wie zum Beispiel der Konzentration oder Aktivität eines Analyten, erfolgt vielfach, indem ein Teil einer Probe mit einem oder mehreren Testreagenzien in einem Reaktionsgefäß, welches auch die Messzelle sein kann, vermischt wird, wodurch z.B. eine biochemische Reaktion oder eine spezifische Bindungsreaktion in Gang gesetzt wird, die eine messbare Veränderung einer optischen oder anderen physikalischen Eigenschaft des Testansatzes bewirkt.

In automatisch arbeitenden Analysatoren, die zur Untersuchung von biologischen Körperflüssigkeiten eingesetzt werden, werden beispielsweise die benötigten Reagenzien mittels einer Pipettiervorrichtung mit einer Pipettiernadel in eine Messküvette eingefüllt. Die Messküvette wird dabei mit einem Küvettengreifer innerhalb des automatischen Analysegerätes zu verschiedenen Positionen mittels eines Robotorarms automatisch verfahren, der Teil einer Robotorstation ist. Nach der Messung wird die benutzte Messküvette zur Entsorgung durch einen Abfallschacht in einen Abfallbehälter verbracht.

In automatischen Analysatoren werden dabei Flüssigkeiten häufig in Kleinstmengen transportiert. Diese geschieht z.B. durch motorisch verschiebbare Pipetten, wobei die Pipetten dabei mittels motorisch betriebener Pumpen betrieben werden. Die Pumpe erzeugt einen definierten Überdruck bei der Flüssigkeitsabgabe und einen definierten Unterdruck bei der Flüssigkeitsaufnahme. Die Pipette ist mit einer inkompressiblen Systemflüssigkeit gefüllt, um die durch die Pumpe vorgegebenen Druckverhältnisse bzw. Druckänderungen möglichst verlustarm an der Pipettenspitze abzubilden und dadurch eine hohe Pipettierpräzision zu ermöglichen.

In medizintechnischen Geräten wie z.B. diagnostischen Analyzern zur automatischen Analyse von in-vitro Proben können Fehler bei der Dosierung der Flüssigkeiten und der Tropfenabgabe durch die Pipetten zu Verfälschungen von Messungen und unrichtigen Messergebnissen führen. Solche Fehler bei der Topfenabgabe entstehen z.B. durch fehlerhafte Dosierung an den Dosiereinheiten.

Zur Überwachung und nachträglichen Verifizierung wurde bisher üblicherweise bei automatischen Analyzern mittels kapazitiver Messungen während der Abgabe der zu dosierenden Flüssigkeit durch den Pipettor, bzw. beim Eintauchen der Pipettiernadel des Pipettors in die pipettierte Flüssigkeit die pipettierte Flüssigkeitsmenge ermittelt, um eventuelle Abweichungen im Füllstand durch z.B. Fehlfunktion der Dosiereinheit zu erkennen. Alternativ wurden Druckmessungen während der Abgabe der zu dosierenden Flüssigkeit durch den Pipettor durchgeführt.

Bei Dosierungen von z.B. Reagenzien an eine schräge Wandung in ein Gefäß durch einen Pipettor zur optimalen Abgabe kleinster Mengen Flüssigkeit kann es passieren, dass es keinen kontrollierten Flüssigkeitsabriss gibt und ein Teil oder die gesamte Menge der Flüssigkeit an der Pipettiernadel haften bleibt und nicht ordnungsgemäß abgegeben wird. Auch eine kapazitive Messung oder Druck-Messung ist diesbezüglich schwer möglich, da etwa mangels Masse eine Gegenelektrode fehlt. An der schrägen Wand läuft die Flüssigkeit ab und ist somit nicht mehr in Kontakt mit dem Pipettor. Bei einmaligen Dosiervorgängen ist daher bisher keine anschließende Überprüfung des geänderten Füllstandes möglich und somit keine Verifikation der korrekten Funktion der Dosiereinheit.

Verfahren zur Überwachung einer Dosierung sind u.a. aus den Dokumenten US 5 601 980 A, US 2018/348247 A1 und US 2019/151840 A1 bekannt.

Die Verfahren aus dem Stand der Technik ermöglichen daher nicht immer eine verlässliche Überwachung der Dosierung von pipettierten Flüssigkeiten in automatischen Analysegeräten.

Es ist daher Aufgabe der Erfindung, ein verbessertes Verfahren zur Uberwachung einer Dosierung einer pipettierten Flüssigkeit für ein automatisches Analysegerät zur Verfügung zu stellen.

**Diese Aufgabe wird erfindungsgemäß durch** das Verfahren nach Anspruch 1 gelöst.

Die Dosierung selbst wird üblicherweise mittels entsprechend automatisierter Laufkontrolle des Antriebsmotors des Kolbens eines entsprechenden Pumpsystems überwacht. Damit ist allerdings z.B. nicht erkennbar, ob tatsächlich das komplette zu pipettierende Volumen der Flüssigkeit z.B. in ein Reaktionsgefäß abgegeben wurde und die Dosierung ordnungsgemäß durchgeführt wurde, oder noch eine signifikante Restmenge der Flüssigkeit z.B. an der Spitze der Pipettiernadel haften bleibt. Eine optische Überwachung hat dabei den Vorteil, dass der Dosierprozess in großer Präzision überwacht werden kann. Dabei erfolgt die Überwachung kontaktfrei und unabhängig von dem Dosierprozess, so dass Störgrößen und Ungenauigkeiten, die z.B. durch Lamellenabriss beim Auftauchen der Pipettiernadel aus Flüssigkeit bei kapazitiver Füllstandsmessung vermieden werden. Bei Dosierprozessen, bei denen nur einmalig eine Flüssigkeitsmenge abgegeben wird und bei denen keine kapazitive Messung möglich ist, da es sich z. B. bei dem Umgebungsmedium nicht um Flüssigkeit handelt, ermöglicht die erfindungsgemäße Vorrichtung erstmals eine präzise Überwachung des tatsächlich durchgeführten Dosierprozesses. Ähnliche Vorteile ergeben sich für die Überwachung von Dosierprozessen, bei denen Flüssigkeit an eine schräge Wandung abgegeben wird und bei denen Topfen der Flüssigkeit an der Wandung abrutschen können.

Zunächst werden bevorzugte Merkmale der im Verfahren benutzte Vorrichtung beschrieben.

Bevorzugt umfasst die die Auswertevorrichtung ein oder mehrere Field Programmable Gate Arrays (FPGAs) und/oder einen Computer, wobei der Computer bevorzugt eine oder mehrere Grafikkarten für die Bildverarbeitung umfasst.

Bevorzugt ist die Kamera mit einem Computer und/oder einen FPGA und/oder einen Mikrocontroller oder einer sonstigen, geeigneten datenverarbeitende Maschine verbunden.

Bevorzugt benutzt das erfindungsgemäße Verfahren eine Triggervorrichtung, die ein Triggersignal an die Kamera zum Erfassen eines Bildes des Tropfens der Flüssigkeit und/oder an die Auswertevorrichtung zur Charakterisierung des Tropfens der Flüssigkeit übermitteln kann. Bevorzugt kann eine kontinuierliche oder quasikontinuierliche Bildaufzeichnung bzw. Auswertung durch das Triggersignal gestartet werden. Bevorzugt umfasst die Triggervorrichtung eine Lichtschranke, z.B. eine Gabellichtschranke und/oder einen Abstandssensor, wobei der Abstandssensor einen Abstand bevorzugt mittels einer time of flight Messung und/oder Triangulation ermittelt. Bevorzugt kann es sich bei dem Abstandssensor auch um einen Ultraschallabstandssensor, einen induktiven Abstandssensor und/oder einen Abstandssensor basierend auf einem variablem Lichtstrom handeln.

Bevorzugt kann das Triggersignal mittels der Triggervorrichtung in Abhängigkeit einer Bewegung einer Vorrichtung ausgelöst werden. Bei der Vorrichtung kann es sich z.B. um eine Küvette und/oder die Pipettiernadel handeln. Bevorzugt ist die Vorrichtung Teil eines automatischen Analysegeräts.

In weiterer bevorzugter Ausführungsform kann das Triggersignal z.B. auch über eine Vorrichtung generiert werden, die ihrerseits eine Bewegung einer anderen Vorrichtung steuert.

Bevorzugt umfasst die Triggervorrichtung z.B. ein elektronisches und/oder optisches Bauelement zum Auslösen eines Vorgangs, bevorzugt eines Schaltvorgangs.

In einer bevorzugten Ausführung umfasst die Beleuchtungsvorrichtung eine Ringbeleuchtung.

In einer weiteren bevorzugten Ausführung ist die Ringbeleuchtung an der Kamera und/oder der Optik angeordnet.

In einer weiteren bevorzugten Ausführung umfasst die Beleuchtungsvorrichtung einen Spiegel. Bevorzugt ist der Spiegel so angeordnet, dass der Tropfen der Flüssigkeit über den Spiegel mittels der Optik der Kamera abgebildet werden kann.

In einer weiteren bevorzugten Ausführung umfasst die Beleuchtungsvorrichtung einen Strahlteiler. Bevorzugt ist der Strahlteiler so angeordnet, dass der Tropfen der Flüssigkeit durch den Strahlteiler mittels der Optik der Kamera abgebildet werden kann. Vorteilhafterweise erfolgt dabei eine Einkopplung der Beleuchtung des Tropfens der Flüssigkeit durch die Beleuchtungsvorrichtung mittels des Strahlteilers.

In einer weiteren bevorzugten Ausführung umfasst die Beleuchtungsvorrichtung mindestens eine Lichtquelle, bevorzugt mehr als eine Lichtquelle, besonders bevorzugt drei Lichtquellen.

Bevorzugt erfolgt die Erfassung des Bildes des Tropfens ausgelöst durch ein Triggersignal einer Triggervorrichtung, welches von der Triggervorrichtung an die Kamera übermittelt wird. Alternativ kann die Erfassung auch unabhängig eines Triggersignals durchgängig erfolgen.

Bevorzugt erfolgt die Charakterisierung des Tropfens mittels der Auswertevorrichtung ausgelöst durch ein Triggersignal einer Triggervorrichtung, welches von der Triggervorrichtung an die Auswertevorrichtung übermittelt wird. Alternativ kann die Charakterisierung des Tropfens auch unabhängig eines Triggersignals durchgängig erfolgen.

Bevorzugt wird eine kontinuierliche oder quasikontinuierliche Bildaufzeichnung bzw. Auswertung durch das Triggersignal gestartet. Die Bildaufzeichnung bzw. Auswertung erfolgt dabei bevorzugt, bis ein weiteres Triggersignal eine entsprechende Beendigung auslöst, oder bis zum Ablauf einer vorbestimmten Zeitspanne.

Bevorzugt wird das Triggersignal mittels der Triggervorrichtung in Abhängigkeit einer Bewegung einer Vorrichtung ausgelöst. Bei der Vorrichtung kann es sich z.B. um eine Küvette und/oder die Pipettiernadel handeln. Bevorzugt ist die Vorrichtung Teil eines automatischen Analysegeräts.

In weiterer bevorzugter Ausführungsform wird das Triggersignal über eine Vorrichtung generiert, die ihrerseits eine Bewegung einer anderen Vorrichtung steuert.

In einer bevorzugten Ausführung des Verfahrens umfasst die Charakterisierung des Tropfens der Flüssigkeit die Bestimmung der Kontur des Tropfens.

In einer weiteren bevorzugten Ausführung des Verfahrens umfasst die Charakterisierung des Tropfens der Flüssigkeit die Bestimmung des Volumens des Tropfens.

In einer weiteren bevorzugten Ausführung des Verfahrens umfasst die Bestimmung des Volumens des Tropfens der Flüssigkeit mindestens eine Annahme über die Symmetrie des Tropfens. Bevorzugt wird dabei eine Symmetrie des Tropfens um mindestens eine Rotationsache angenommen.

Falls die Dosierung nicht ordnungsgemäß verlaufen ist, wird vorteilshafterweise automatisch eine entsprechende Kennzeichnung des Dosiervorgangs vorgenommen und z.B. dem Laborpersonal etwa über eine entsprechende Anzeige auf einem Monitor oder auf einem Papierausdruck zur Kenntnis gebracht. Alternativ kann ein entsprechender Messvorgang auch z.B. automatisch abgebrochen und neu gestartet werden.

Alternativ wird vorteilhafterweise die erfasste Flüssigkeitsmenge des Tropfens als Korrekturwert herangezogen und die tatsächlich erfolgte Dosierung wird ermittelt, indem von einer geplante Dosierung die erfasste Flüssigkeitsmenge des Tropfens abgezogen wird. Optional kann dann vorteilshafterweise auch eine entsprechende weitere Dosierung erfolgen, die die noch fehlende Flüssigkeitsmenge hinzufügt. Dies hat den Vorteil, dass Fehlmessungen vermieden werden und/oder als solche erkennbar sind.

In einer bevorzugten Ausführung des Verfahrens erfolgt die Bestimmung, ob die Dosierung der Flüssigkeit ordnungsgemäß verlaufen ist, und/oder der Güte der Dossierung mittels maschinellem Lernen und/oder oder umfasst einen Einsatz eines Machine-Learning-Systems.

In einer bevorzugten Ausführungsform wird das Volumen des an der Pipettiernadel haftenden Tropfens zunächst durch Auswiegen der entsprechenden Flüssigkeitsmenge und anschließenden Abgleich mittels Bilddaten für eine Vielzahl von Tropfen bestimmt. Die Bilddaten umfassen dabei die Bilder der Tropfen. Bevorzugt erfolgt also eine vorhergehende Kalibration. Bevorzugt erfolgt anschließend mittels maschinellem Lernens eine entsprechende Zuordnung einer Güte der Dosierung der Flüssigkeit.

Ein weiterer Gegenstand der Erfindung ist die Verwendung des erfindungsgemäße Verfahrens zur optischen Überwachung einer Dosierung einer zu pipettierenden Flüssigkeit in einem automatischen Analysegeräts, wobei das automatische Analysegerät bevorzugt einen automatischen Küvettengreifer und/oder einen automatischen Pipettor umfasst.

Unter einer "Probe" ist im Sinne der Erfindung das Material zu verstehen, dass die nachzuweisende Substanz (den Analyten) vermutlich enthält. Der Begriff "Probe" umfasst insbesondere biologische Flüssigkeiten von Menschen oder Tieren wie z.B. Blut, Plasma, Serum, Sputum, Exsudat, bronchoalveoläre Lavage, Lymphflüssigkeit, Synovialflüssigkeit, Samenflüssigkeit, Vaginalschleim, Feces, Urin, Liquor, aber auch z.B. durch Homogenisation oder Zelllyse für die photometrische, bevorzugt nephelometrische Bestimmung entsprechend aufgearbeitete Gewebe- oder Zellkulturproben. Ferner können auch z.B. pflanzliche Flüssigkeiten oder Gewebe, forensische Proben, Wasser- und Abwasserproben, Nahrungsmittel, Arzneimittel als Probe dienen, die ggf. vor der Bestimmung einer entsprechenden Probenvorbehandlung zu unterziehen sind.

Bei einem quantitativen Nachweis wird die Menge, die Konzentration oder die Aktivität des Analyten in der Probe gemessen. Von dem Begriff des "quantitativen Nachweises" sind auch semiquantitative Methoden umfasst, die nur die ungefähre Menge, Konzentration oder Aktivität des Analyten in der Probe erfassen oder nur zu einer relativen Mengen-, Konzentrations- oder Aktivitätsangabe dienen können. Unter einem qualitativen Nachweis ist der Nachweis des Vorhandenseins des Analyten in der Probe überhaupt oder das Anzeigen, dass die Menge, Konzentration oder Aktivität des Analyten in der Probe unterhalb oder oberhalb eines bestimmten oder mehrerer bestimmter Schwellenwerte liegt, zu verstehen.

Bei einer Messküvette handelt es sich beispielsweise um eine Küvette oder ein Reaktionsgefäß aus Glas, Kunststoff oder Metall. Vorteilhafterweise ist die Messküvette aus optisch transparenten Materialien gefertigt, was besonders beim Einsatz von optischen Analyseverfahren vorteilhaft sein kann.

Die Begriffe "Messküvette" und "Küvette" werden synonym verwendet und bezeichnen den gleichen Gegenstand.

Die Begriffe "Analysegerät" und "Analyzer" werden vorliegend synonym verwendet und bezeichnen den gleichen Gegenstand.

Bei einem Topfen einer Flüssigkeit handelt es sich um eine Menge der Flüssigkeit, die z.B. in Form eines Flüssigkeitsfilms, in Formen ähnlich der Form von Birnen oder als Flüssigkeitskügelchen in Kugelform vorliegen kann. Bei der Menge der Flüssigkeit handelt es sich bevorzugt z.B. um kleinere Flüssigkeitsmengen, die z.B. im Bereich von 1 bis 100 Microlitern, bevorzugt im Bereich 5 bis 10 Microlitern liegen können.

Die Kamera umfasst bevorzugt ein digitales Aufzeichnungsgerät umfassend einen charge-coupled device (CCD) Chip oder mehrere CCD Chips. Besonders bevorzugt basiert das digitale Aufzeichnungsgerät auf einer Complementary Metal-Oxide-Semiconductor (CMOS) Technik und/oder umfasst einen CMOS Chip. Bei der Kamera kann es sich bevorzugt auch um ein digitales Aufzeichnungsgerät handeln.

**Die Erfindung wird anhand von Zeichnungen exemplarisch näher erläutert. Darin zeigen:**

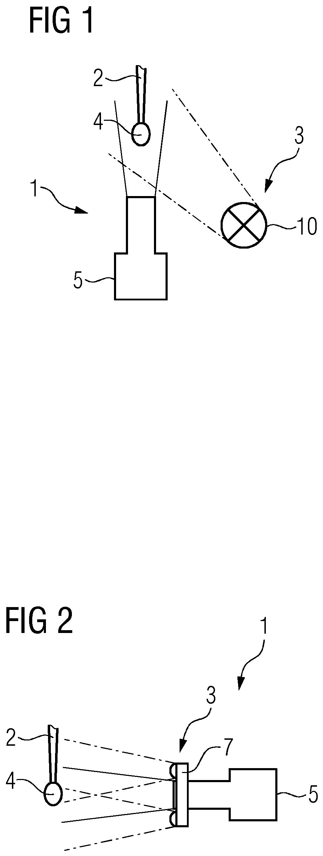

FIG 1, 2, 3 und 4 schematisch den Aufbau unterschiedlicher Vorrichtungen zur optischen Überwachung einer Dosierung einer zu pipettierenden Flüssigkeit für ein automatisches Analysegerät.

### Gleiche Teile sind in allen Figuren mit denselben Bezugszeichen versehen.

Die Vorrichtung (1) gemäß FIG 1 bis 4 ist eingebettet in ein nicht näher dargestelltes Analysegerät, welches zur Ausführung einer Vielzahl von Analysen von Proben ausgestaltet ist. Dazu umfasst das automatische Analysegerät eine Vielzahl von nicht gezeigten Pipettiereinrichtungen und Transporteinrichtungen sowie weiterhin eine Steuereinheit zum automatisierten Auswerten der Analysen und eine Auswertevorrichtung (6) zur Charakterisierung eines an der Dosiervorrichtung (2) haftenden Tropfens (4) einer Flüssigkeit mittels einer automatischen Auswertung eines Bildes des Tropfens (4) der Flüssigkeit. Die Vorrichtungen (1) sind jeweils ausgestaltet zur optischen Überwachung einer Dosierung einer zu pipettierenden Flüssigkeit für ein automatisches Analysegerät.

Bei der in FIG 1 gezeigten Vorrichtung (1) wird mittels der Dosiervorrichtung (2) ein Tropfen (4) einer Flüssigkeit pipettiert. Der Tropfen (4) haftet an der Spitze einer Pipettiernadel. Unterhalb des Tropfens (4) ist eine Kamera (5) mit einer Optik angeordnet. Der Tropfen wird von schräg unten mittels einer Beleuchtungsvorrichtung (3) direkt beleuchtet, wobei die Beleuchtungsvorrichtung (3) eine Lichtquelle (10) umfasst.

Bei der in FIG 2 gezeigten Vorrichtung (1) wird mittels der Dosiervorrichtung (2) ein Tropfen (4) einer Flüssigkeit pipettiert. Der Tropfen (4) haftet an der Spitze einer Pipettiernadel. Auf Höhe des Tropfens (4) ist eine Kamera (5) mit einer Optik seitlich angeordnet. Der Tropfen wird seitlich mittels einer Beleuchtungsvorrichtung (3) beleuchtet, die als Ringbeleuchtung (7) ausgestaltet ist. Die Ringbeleuchtung (7) ist an der Optik der Kamera (5) angeordnet.

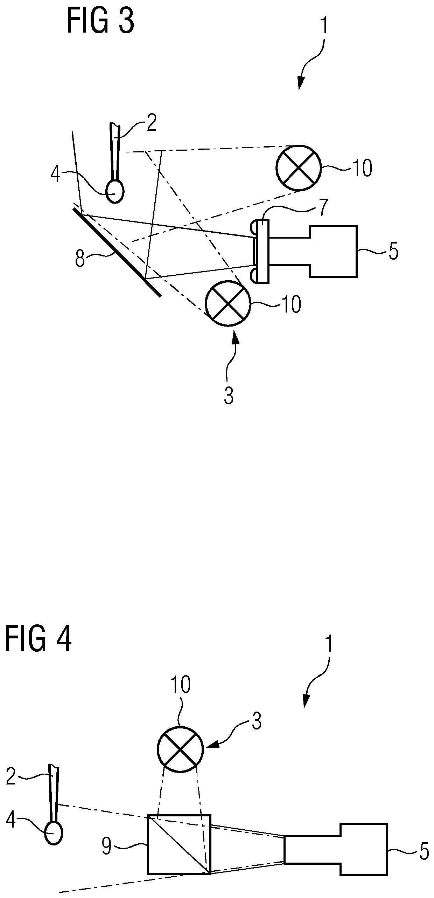

Bei der in FIG 3 gezeigten Vorrichtung (1) wird mittels der Dosiervorrichtung (2) ein Tropfen (4) einer Flüssigkeit pipettiert. Der Tropfen (4) befindet sich an der Spitze einer Pipettiernadel. Etwas unterhalb der Höhe des Tropfens (4) ist eine Kamera (5) mit einer Optik seitlich angeordnet. Der Tropfen wird seitlich mittels einer Beleuchtungsvorrichtung (3) beleuchtet, die eine Ringbeleuchtung (7), die an der Optik der Kamera (5) angeordnet ist umfasst sowie zwei weitere Lichtquellen (10).

Sowohl die Ringbeleuchtung (7) als auch die zwei weiteren Lichtquellen (10) beleuchten den Tropfen (10) direkt. Weiter ist ein optischer Spiegel (8) unterhalb des Tropfens (4) in einem Winkel zur optischen Ache der Kamera (5) angeordnet. Die Abbildung des Tropfens durch die Optik der Kamera (5) erfolgt über den Spiegel (8).

Bei der in FIG 4 gezeigten Vorrichtung (1) wird mittels der Dosiervorrichtung (2) ein Tropfen (4) einer Flüssigkeit pipettiert. Der Tropfen (4) haftet an der Spitze einer Pipettiernadel. Auf der Höhe des Tropfens (4) ist eine Kamera (5) mit einer Optik seitlich angeordnet. Zwischen Tropfen (4) und Kamera (5) ist ein Strahlteiler (9) angeordnet. Oberhalb des Strahlteilers (9) ist eine Beleuchtungsvorrichtung (3) vorgesehen, die eine Lichtquelle (10) umfasst. Das von der Lichtquelle ausgehende Licht wird mittels des Strahlteilers (9) umgelenkt und trifft entlang der optischen Ache der Kamera (5) auf den Tropfen (4) und beleuchtet diesen. Die Abbildung des Tropfens (4) erfolgt durch den Strahlteiler (9) hindurch mittels der Optik der Kamera.

### Bezugszeichenliste

- 1: Vorrichtung
- 2: Dosiervorrichtung
- 3: Beleuchtungsvorrichtung
- 4: Tropfen
- 5: Kamera
- 6: Auswertevorrichtung
- 7: Ringbeleuchtung
- 8: Spiegel
- 9: Strahlteiler
- 10: Lichtquelle

## Patentansprüche

1. Verfahren zur optischen Überwachung einer Dosierung einer zu pipettierenden Flüssigkeit für ein automatisches Analysegerät mittels Erfassung eines Bildes eines Tropfens (4) der Flüssigkeit mithilfe einer Vorrichtung (1) zur optischen Überwachung einer Dosierung einer zu pipettierenden Flüssigkeit für ein automatisches Analysegerät, die Vorrichtung umfassend
eine Dosiervorrichtung (2) umfassend eine Pipettiernadel zur Pipettierung der Flüssigkeit,
eine Beleuchtungsvorrichtung (3) zur Beleuchtung eines an der Pipettiernadel haftenden Tropfens (4) der Flüssigkeit,
eine Kamera (5) mit einer Optik zum Erfassen eines Bildes des Tropfens (4) der Flüssigkeit,
eine Auswertevorrichtung (6) zur Charakterisierung des Tropfens (4) der Flüssigkeit mittels einer automatischen Auswertung des Bildes des Tropfens (4) der Flüssigkeit, das Verfahren umfassend die folgenden Schritte:
- Dosierung der Flüssigkeit durch Pipettierung mittels der Dosiervorrichtung (2), wobei eine Restmenge der Flüssigkeit in Form eines Tropfens (4) an der Spitze der Pipettiernadel nach der Dosierung haften bleibt,
- Beleuchtung des nach Abschluss der Dosierung an der Pipettiernadel haftenden Tropfens (4) der Flüssigkeit mittels der Beleuchtungsvorrichtung (3),
- Erfassen des Bildes des Tropfens (4) der Flüssigkeit mittels der Optik und der Kamera (5),
- Ermittlung der Flüssigkeitsmenge des Tropfens (4) mittels der Auswertevorrichtung (6) und der automatischen Auswertung des Bildes des Tropfens (4) der Flüssigkeit, und
- Ermittlung der dosierten Flüssigkeitsmenge als der Differenz zwischen der geplanten zu dosierenden Flüssigkeitsmenge und der Flüssigkeitsmenge des Tropfens (4) oder
- wobei anhand der ermittelten Flüssigkeitsmenge des Tropfens (4) bestimmt wird, ob die Dosierung der Flüssigkeit ordnungsgemäß verlaufen ist und/oder eine Güte der Dosierung bestimmt wird, indem die ermittelte Flüssigkeitsmenge des Tropfens (4) mit einem vorbestimmten absoluten Grenzwert, der von der Menge der zu dosierenden Flüssigkeitsmenge abhängt, verglichen wird, wobei die Bestimmung bevorzugt mittels der Auswertevorrichtung (6) erfolgt.

2. Verfahren nach Anspruch 1, wobei die Beleuchtungsvorrichtung eine Ringbeleuchtung (7) umfasst.

3. Verfahren nach Anspruch 2, wobei die Ringbeleuchtung (7) an der Kamera (5) und/oder der Optik angeordnet ist.

4. Verfahren nach einem der vorhergehenden Ansprüche, wobei die Beleuchtungsvorrichtung einen Spiegel (8) umfasst.

5. Verfahren nach einem der vorhergehenden Ansprüche, wobei die Beleuchtungsvorrichtung einen Strahlteiler (9) umfasst.

6. Verfahren nach einem der vorhergehenden Ansprüche, wobei die Beleuchtungsvorrichtung mindestens drei Lichtquellen (10) umfasst.

7. Verfahren nach einem der vorhergehenden Ansprüche, wobei die Charakterisierung des Tropfens (4) der Flüssigkeit die Bestimmung der Kontur des Tropfens (4) umfasst.

8. Verfahren nach einem der vorhergehenden Ansprüche, wobei die Charakterisierung des Tropfens (4) der Flüssigkeit die Bestimmung des Volumens des Tropfens (4) umfasst.

9. Verfahren nach Anspruch 8, wobei die Bestimmung des Volumens des Tropfens (4) der Flüssigkeit mindestens eine Annahme über die Symmetrie des Tropfens (4) umfasst, wobei bevorzugt Symmetrie des Tropfens um mindestens eine Rotationsache angenommen wird.

10. Verfahren nach einem der vorhergehenden Ansprüche, wobei mittels der Charakterisierung des Tropfens (4) der Flüssigkeit eine kontaktfreie Erfassung der Flüssigkeitsmenge des Tropfens (4) erfolgt.

11. Verfahren nach Anspruch 1, wobei die Bestimmung, ob die Dosierung der Flüssigkeit ordnungsgemäß verlaufen ist und/oder die Bestimmung der Güte der Dosierung mittels maschinellem Lernen erfolgt und/oder einen Einsatz eines Machine-Learning-Systems umfasst.

12. Verwendung eines Verfahrens nach einem der Ansprüche 1 bis 11 in einem automatischen Analysegerät, wobei das automatische Analysegerät bevorzugt einen automatischen Küvettengreifer und/oder einen automatischen Pipettor umfasst.

## Claims

1. Method for optically monitoring a dosing of a liquid to be pipetted for an automatic analysis unit by acquiring an image of a drop (4) of the liquid with the aid of an apparatus (1) for optically monitoring a dosing of a liquid to be pipetted for an automatic analysis unit, the apparatus comprising a dosing device (2) comprising a pipetting needle for pipetting the liquid,
a lighting device (3) for illuminating a drop (4) of the liquid adhering to the pipetting needle,
a camera (5) with a set of optics for acquiring an image of the drop (4) of the liquid,
an evaluation device (6) for characterizing the drop (4) of the liquid by means of an automatic analysis of the image of the drop (4) of the liquid, the method comprising the following steps:
- dosing of the liquid by pipetting using the dosing device (2), wherein a residual amount of the liquid in the form of a drop (4) remains stuck to the tip of the pipetting needle after the dosing,
- using the lighting device (3) to illuminate the drop (4) of the liquid adhering to the pipetting needle after the dosing is completed,
- acquiring the image of the drop (4) of the liquid using the set of optics and the camera (5),
- establishing the quantity of liquid in the drop (4) by means of the evaluation device (6) and automatically evaluating the image of the drop (4) of the liquid, and
- establishing the dosed quantity of liquid as the difference between the planned quantity of liquid to be dosed and the quantity of liquid in the drop (4), or
- the established quantity of liquid in the drop (4) being used to determine whether the dosing of the liquid has been performed correctly and/or to determine a quality of the dosing by comparing the established quantity of liquid in the drop (4) with a predetermined absolute limit value which depends on the amount of the quantity of liquid to be dosed, the determination preferably being carried out by means of the evaluation device (6).

2. Method according to Claim 1, wherein the lighting device comprises a ring illuminator (7).

3. Method according to Claim 2, wherein the ring illuminator (7) is located on the camera (5) and/or the set of optics.

4. Method according to any of the preceding claims, wherein the lighting device comprises a mirror (8).

5. Method according to any of the preceding claims, wherein the lighting device comprises a beam splitter (9).

6. Method according to any of the preceding claims, wherein the lighting device comprises at least three light sources (10).

7. Method according to any of the preceding claims, wherein the characterization of the drop (4) of the liquid comprises the determination of the outline of the drop (4).

8. Method according to any of the preceding claims, wherein the characterization of the drop (4) of the liquid comprises the determination of the volume of the drop (4).

9. Method according to Claim 8, wherein the determination of the volume of the drop (4) of the liquid comprises at least one assumption about the symmetry of the drop (4), wherein symmetry of the drop about at least one rotational axis is preferably assumed.

10. Method according to any of the preceding claims, wherein by means of the characterization of the drop (4) of the liquid the quantity of liquid in the drop (4) is detected in a contactless manner.

11. Method according to Claim 1, wherein the determination whether the dosing of the liquid has been performed correctly and/or the determination of the quality of the dosing is carried out by machine learning and/or comprises the use of a machine learning system.

12. Use of a method according to any of Claims 1 to 11 in an automatic analysis unit, said automatic analysis unit preferably comprising an automatic cuvette gripper and/or an automatic pipettor.

## Revendications

1. Procédé de contrôle optique d'un dosage d'un liquide à pipetter pour un appareil d'analyse automatique, au moyen de la prise d'une image d'une goutte (4) du liquide, à l'aide d'un dispositif (1) de contrôle optique d'un dosage d'un liquide à pipetter pour un appareil d'analyse automatique, le dispositif comprenant
un dispositif (2) de dosage comprenant une aiguille de pipetage pour le pipetage du liquide,
un dispositif (3) d'éclairage pour l'éclairage d'une goutte (4) du liquide adhérent à l'aiguille de pipetage,
un appareil (5) photographique ayant une optique pour prendre une image de la goutte (4) du liquide,
un dispositif (6) d'évaluation pour la caractérisation de la goutte (4) du liquide, au moyen d'une évaluation automatique de l'image de la goutte (4) du liquide, le procédé comprenant les stades suivants :
- dosage du liquide par pipetage, au moyen du dispositif (2) de dosage, dans lequel une quantité résiduelle du liquide reste adhérente sous la forme d'une goutte (4) à la pointe de l'aiguille de pipetage après le dosage,
- éclairage, au moyen du dispositif (3) d'éclairage, de la goutte (4) de liquide restant sur l'aiguille de pipetage après la fin du dosage,
- prise de l'image de la goutte (4) du liquide au moyen de l'optique et de l'appareil (5) photographique,
- détermination de la quantité de liquide de la goutte (4) au moyen du dispositif (6) d'évaluation et de l'évaluation automatique de l'image de la goutte (4) du liquide, et
- détermination de la quantité de liquide dosée comme différence entre la quantité de liquide planifiée à doser et la quantité de liquide de la goutte (4) ou
- dans lequel, à l'aide de la quantité déterminée de liquide de la goutte (4), on détermine si le dosage du liquide s'est dûment déroulé et/ou on détermine une qualité du dosage, en comparant la quantité déterminée de liquide de la goutte (4) à une valeur limite absolue déterminée à l'avance, qui dépend de la quantité de liquide à doser, dans lequel la détermination s'effectue, de préférence, au moyen du dispositif (6) d'évaluation.

2. Procédé suivant la revendication 1, dans lequel le dispositif d'éclairage comprend un éclairage (7) annulaire.

3. Procédé suivant la revendication 2, dans lequel l'éclairage (7) annulaire est monté sur l'appareil (5) photographique et/ou sur l'optique.

4. Procédé suivant l'une des revendications précédentes, dans lequel le dispositif d'éclairage comprend un miroir (8).

5. Procédé suivant l'une des revendications précédentes, dans lequel le dispositif d'éclairage comprend un diviseur (9) de faisceau.

6. Procédé suivant l'une des revendications précédentes, dans lequel le dispositif d'éclairage comprend au moins trois sources (10) lumineuses.

7. Procédé suivant l'une des revendications précédentes, dans lequel la caractérisation de la goutte (4) du liquide comprend la détermination du contour de la goutte (4).

8. Procédé suivant l'une des revendications précédentes, dans lequel la caractérisation de la goutte (4) du liquide comprend la détermination du volume de la goutte (4).

9. Procédé suivant la revendication 8, dans lequel la détermination du volume de la goutte (4) du liquide comprend au moins une présomption sur la symétrie de la goutte (4), dans lequel une symétrie préférée de la goutte, autour d'au moins un axe de révolution, est présumée.

10. Procédé suivant l'une des revendications précédentes, dans lequel une détection sans contact de la quantité de liquide de la goutte (4) s'effectue au moyen de la caractérisation de la goutte (4) du liquide.

11. Procédé suivant la revendication 1, dans lequel la détermination, si le dosage du liquide est dûment effectué et/ou la détermination de la qualité du dosage s'effectue au moyen d'un apprentissage par machine et/ou comprend l'utilisation d'un système d'apprentissage par machine.

12. Utilisation d'un procédé suivant l'une des revendications 1 à 11, dans un appareil d'analyse automatique, dans lequel l'appareil d'analyse automatique comprend, de préférence, un dispositif automatique de préhension de cuvette et/ou un pipeteur automatique.
